# EUROPÄISCHE PATENTANMELDUNG

(11) **EP 2 478 933 A2**
(43) Veröffentlichungstag der Anmeldung: **25.07.2012**
(21) Anmeldenummer: 11192603.6
(22) Anmeldetag: 08.12.2011
(51) Int. Cl.: A61N 1/08

(54) **Implantierbares Gerät**

(30) Priorität: 20.12.2010 US 201061424690 P
(71) Anmelder: BIOTRONIK SE & Co. KG, 12359 Berlin (DE)
(72) Erfinder: Bartels, Klaus, 10115 Berlin (DE); Frenzel, Timo, 12435 Berlin (DE); Knorr, Stefan, 10119 Berlin (DE)
(74) Vertreter: Lindner-Vogt, Karin L.

(57) **Zusammenfassung**

Die Erfindung betrifft ein temporär oder permanent implantierbares medizinisches Gerät, mit mindestens einer langgestreckten elektrischen Leitung , die einen Funktionsleiter enthält, welcher mit einem Elektrodenpol zur Abgabe von Therapiesignalen oder zur Erfassung von Diagnosesignalen verbunden ist, wobei der Funktionsleiter oder der Elektrodenpol, oder sowohl der Funktionsleiter als auch der Elektrodenpol in einem ersten Längsabschnitt ringförmig ausgebildet ist; und die mindestens einen zweiten elektrischen Leiter enthält, der in dem ersten Längsabschnitt wendelförmig geführt ist, derart, dass im ersten Leiter führbare elektromagnetische Radiofrequenz-Wellen zumindest teilweise im ersten Längsabschnitt in den zweiten elektrischen Leiter einkoppelbar sind.

## Beschreibung

Die Erfindung betrifft ein permanent oder temporär implantierbares medizinisches Gerät mit einem langgestreckten elektrischen Leiter.

Solche Geräte, beispielsweise Elektrodenleitungen für die Elektrostimulation, haben den Nachteil, dass sich ihr elektrischer Leiter in einem Kernspintomografen erwärmen kann, weil die im Kernspintomografen herrschenden wechselnden Magnetfelder in dem elektrischen Leiter nicht unbeachtliche elektrische Ströme induzieren. Deshalb können Herzschrittmacherpatienten heutzutage in der Regel nicht oder nur eingeschränkt in einem Kernspintomografen untersucht werden.

An implantierbaren Herzschrittmachern oder Defibrillatoren sind nämlich typischerweise wenigstens eine Stimulationselektrodenleitung angeschlossen, die an ihrem proximalen, zum Anschluss an den Herzschrittmacher oder Defibrillator vorgesehenen Ende einen standardisierten elektrischen Anschluss aufweist und an ihrem distalen, zur Platzierung im Herzen vorgesehenen Ende einen oder mehrere Elektrodenpole aufweist. Ein solcher Elektrodenpol dient zur Abgabe elektrischer Impulse, beispielsweise an das Gewebe (Myokard) des Herzens oder zum Abfühlen elektrischer Felder, um im Rahmen des sogenannten Sensings eine Aktivität, beispielsweise eine Herzaktivität abfühlen zu können.

Zu diesen Zwecken bilden Elektrodenpole typischerweise elektrisch leitende Oberflächenabschnitte einer Elektrodenleitung. Elektrodenpole sind typischerweise als Ringelektrode in Form eines Rings um die Elektrodenleitung oder in Form einer Spitzen- oder Tippelektrode am distalen Ende der Elektrodenleitung vorgesehen.

Die Elektrodenpole sind über einen oder mehrere elektrische Leiter mit Kontakten des elektrischen Anschlusses der Elektrodenleitung an deren proximalem Ende elektrisch leitend verbunden. Somit verlaufen zwischen den Kontakten des elektrischen Anschlusses die Elektrodenleitungen an deren proximalem Ende und den Elektrodenpolen am distalen Ende der Elektrodenleitung ein oder mehrere elektrische Leiter, die einen oder mehrere der Elektrodenpole mit einem oder mehreren der Kontakte elektrisch verbinden. Diese elektrischen Leiter können einerseits zur Übertragung von Stimulationsimpulsen zu den Elektrodenpolen und andererseits zur Übertragung mittels der Elektrodenpole aufgenommener elektrischer Signale zum proximalen Ende der Elektrodenleitung genutzt werden und werden im Verlauf der weiteren Beschreibung auch jeweils als Funktionsleiter bezeichnet.

Solche Funktionsleiter sind für die Funktionen der jeweiligen Elektrodenleitung erforderliche elektrische Leiter und als solche der Gefahr ausgesetzt, dass in ihnen durch äußere Wechselmagnetfelder elektrische Ströme induziert werden, die beispielsweise zu einer unerwünschten Erwärmung der Funktionsleitungen oder der mit ihr verbundenen Elektrodenpole führen können oder die zur Abgabe entsprechender Ströme über die Elektrodenpole an umgebendes Gewebe und damit zu einer Erwärmung des umgebenden Gewebes führen können.

Der Erfindung liegt die Aufgabe zugrunde, ein medizinisches Gerät zu schaffen, welches das zuvor beschriebene Problem löst.

Erfindungsgemäß wird diese Aufgabe gelöst durch ein temporär oder permanent implantierbares medizinisches Gerät, mit mindestens einer langgestreckten elektrischen Leitung, die einen Funktionsleiter enthält, welcher mit einem Elektrodenpol zur Abgabe von Therapiesignalen oder zur Erfassung von Diagnosesignalen verbunden ist, wobei der Funktionsleiter oder der Elektrodenpol oder sowohl der Funktionsleiter als auch der Elektrodenpol in einem ersten Längsabschnitt ringförmig ausgebildet ist; und die mindestens einen zweiten elektrischen Leiter enthält, der in dem ersten Längsabschnitt wendelförmig geführt ist, derart, dass im ersten Leiter führbare elektromagnetische Radiofrequenz-Wellen zumindest teilweise im ersten Längsabschnitt in den zweiten elektrischen Leiter einkoppelbar sind. Das erfindungsgemäße medizinische Gerät erzielt eine Verringerung der unerwünschten Erwärmung des Funktionsleiters oder eines mit ihm verbundenen Elektrodenpols, die durch elektrische Ströme verursacht werden, welche äußere Wechselmagnetfelder im Funktionsleiter der elektrischen Leitung induzieren können. Auf diese Weise kann eine unerwünschte Erwärmung von Körpergewebe im implantierten Zustand reduziert, zumindest teilweise auf andere Gewebebereiche verlagert oder sogar vollständig vermieden werden. Dies gelingt erfindungsgemäß mit Hilfe eines in dem ersten Längsabschnitt wendelförmigen zweiten elektrischen Leiters und mit Hilfe einer Kopplung zwischen dem ersten und dem zweiten elektrischen Leiter, die ausgebildet ist, im ersten Leiter geführte elektromagnetische Radiofrequenz-Wellen zumindest teilweise in den zweiten elektrischen Leiter einzukoppeln.

Zusätzlich werden durch diesen zusätzlich geführten Leiter die mechanischen Eigenschaften optimiert. Das betrifft beispielsweise eine optimierte Biegefestigkeit und Festigkeit gegenüber äußeren mechanischen Einflüssen wie Quetschung, Knickung, Dehnung, Torsion. Damit sinkt auch die Gefahr eines so genannten "Subclavian Crush".

Nachfolgend werden Ausführungsbeispiele des medizinischen Geräts der vorliegenden Erfindung erläutert. Die zusätzlichen Merkmale der einzelnen Ausführungsbeispiele können miteinander zur Bildung weiterer Ausführungsformen des medizinischen Geräts kombiniert werden, soweit sie nicht ausdrücklich als einander ausschließende Alternativen beschrieben sind.

Der Funktionsleiter oder der Elektrodenpol, oder sowohl der Funktionsleiter als auch der Elektrodenpol sind in einem ersten Längsabschnitt ringförmig ausgebildet. Sowohl der Funktionsleiter als auch der Elektrodenform können die Ringform durch eine wendelförmige Wicklung eines Leiters annehmen. Insbesondere ist in einer Ausführungsform der ringförmig ausgebildete Elektrodenpol von dem im ersten Längsabschnitt unisoliert und wendelförmig geführten Funktionsleiter gebildet ist.

Der zweite elektrische Leiter enthält in bevorzugten Ausführungsbeispielen keinen Funktionsleiter, sondern einen oder mehrere Zusatzleiter.

Bevorzugt sind der Funktionsleiter und der zweite elektrische Leiter auf einer gemeinsamen, gedachten Zylindermantel-Fläche wendelförmig geführt. Der Funktionsleiter und der zweite elektrische Leiter können im ersten Längsabschnitt als von einander elektrisch isoliertes Leiterpaar koradial geführt sein.

In alternativen Ausführungsformen sind der Funktionsleiter und der zweite elektrische Leiter im ersten Längsabschnitt auf Block gewickelt oder auf Lücke gewickelt sind. Die Wicklung auf Lücke zeichnet sich durch eine höhere Kompressibilität in Längsrichtung der elektrischen Leitung aus.

Der Funktionsleiter kann außerhalb des ersten Längsabschnitts entweder wendelförmig oder als Seilzuleitung ausgebildet sein.

Bei einer wendelförmigen Führung des Funktionsleiters hat die elektrische Leitung eine isolierende hohlzylindrische Ummantelung, die im Inneren ein Lumen aufweist, in dem eine Innenwendel des ersten elektrischen Leiters geführt ist. Alternativ kann die Seilzuleitung außerhalb des ersten Längsabschnitts in die Ummantelung eingebettet sein.

Der zweite elektrische Leiter ist in einer Ausführungsform im ersten Längsabschnitt von einer Leiterisolierung umgeben, um etwa im Bereich eines Elektrodenpols eine Abgabe von eingekoppelter Energie aus dem zweiten elektrischen Leiter in das umgebende Körpergewebe so gering wie möglich zu halten. In anderen Längsabschnitten kann der zweite elektrische Leiter Ansonsten teilweise oder vollständige unisoliert ausgeführt werden.

Um die Herstellung dieses Ausführungsbeispiels zu vereinfachen, hat der zweite elektrische Leiter vorteilhafterweise einen anderen Durchmesser hat als der Funktionsleiter. Dies kann durch unterschiedliche Drahtstärken oder Isolationsdurchmesser realisiert werden und vereinfacht ein Abisolieren des zweiten elektrischen Leiters in passiven Bereichen des Elektrodenpols.

Der zweite elektrische Leiter kann in Längsabschnitten außerhalb des ersten Längsabschnitts auf eine Ummantelung der des ersten elektrischen Leiters gewickelt sein. Dabei ist der zweite elektrische Leiter vorzugsweise in einem aktiven Teil, also an einem anderen als einem Funktionselektrodenpol teils unisoloiert, im inaktiven Teil jedoch isoliert.

Der Funktionsleiter und der zweite elektrische Leiter sind in einem anderen Ausführungsbeispiel im ersten Längsabschnitt in eine elektrisch isolierende Ummantelung des ersten elektrischen Leiters eingebettet.

Zur Erhöhung der Kopplung zwischen dem Funktionsleiter bzw. dem Elektrodenpol im ersten Längsabschnitt und dem zweiten elektrischen Leiter erstreckt sich der zweite elektrische Leiter in einer Ausführungsform in proximaler oder distaler Richtung oder sowohl in proximaler als auch in distaler Richtung wendelförmig gewickelt über den ersten Längsabschnitt hinaus.

Die langgestreckte elektrische Leitung ist in bevorzugten Ausführungsbeispielen eine temporär oder permanent implantierbare Elektrodenleitung zur Verbindung eines oder mehrerer Funktions-Elektrodenpole mit einem Steuergerät, wie beispielsweise dem Steuergerät eines implantierbaren Herzschrittmachers oder eines implantierbaren Defibrillators. Die langgestreckte Leitung bildet jedoch als solche schon ein medizinisches Gerät im Sinne der vorliegenden Beschreibung.

Nachfolgend werden weitere Ausführungsbeispiele anhand der Figuren erläutert. Es zeigen:
- Fig. 1: ein Ausführungsbeispiel eines erfindungsgemäßen medizinischen Geräts in Form eines Herzschrittmachers;
- Fig. 2: in ihrer Teilfigur Fig. 2A eine schematische teilweise Schnittansicht einer Elektrodenleitung und Fig. 2B eine Querschnittsansicht der Elektrodenleitung aus Fig. 2A;
- Fig. 3: in ihrer Teilfigur Fig. 3A eine schematische Seitenansicht einer Elektrodenleitung und Fig. 3B eine Querschnittsansicht der Elektrodenleitung aus Fig. 3A;
- Fig. 4: in ihrer Teilfigur Fig. 4A eine schematische Seitenansicht einer Elektrodenleitung und Fig. 4B eine Querschnittsansicht der Elektrodenleitung aus Fig. 4A;
- Fig. 5: in ihrer Teilfigur Fig. 5A eine schematische Seitenansicht einer Elektrodenleitung und Fig. 5B eine Querschnittsansicht der Elektrodenleitung aus Fig. 5A;
- Fig. 6: in ihrer Teilfigur Fig. 6A eine schematische Schnittansicht einer Elektrodenleitung und Fig. 6B eine Querschnittsansicht der Elektrodenleitung aus Fig. 6A;
- Fig. 7: in ihrer Teilfigur Fig. 7A eine schematische teilweise Seitenansicht einer Elektrodenleitung und Fig. 7B eine Querschnittsansicht der Elektrodenleitung aus Fig. 7A;

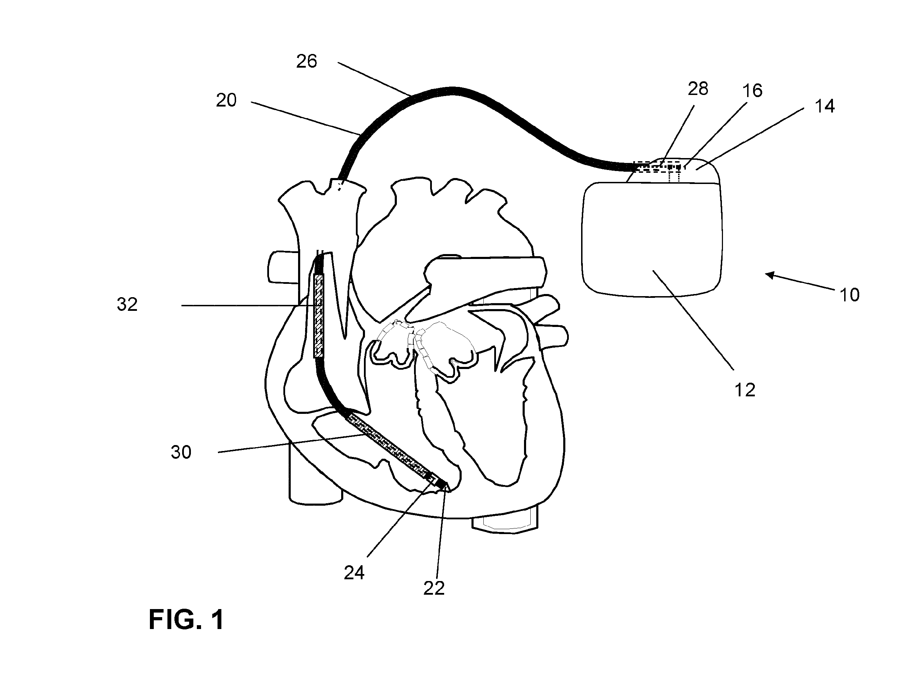

Fig. 1 zeigt als Beispiele implantierbarer medizinischer Geräte einen implantierbaren Herzstimulator 10 und eine daran angeschlossene implantierbare Elektrodenleitung 20.

Der implantierbare Herzstimulator 10 kann ein Herzschrittmacher oder ein Kardioverter/Defibrillator (ICD) sein. Im dargestellten Ausführungsbeispiel ist der Herzstimulator 10 ein ventrikulärer Herzschrittmacher und Defibrillator. Andere bekannte Herzstimulatoren sind Zweikammerherzschrittmacher zur Stimulation des rechten Atriums und des rechten Ventrikels oder biventrikuläre Herzschrittmacher, die zusätzlich zum rechten Ventrikel auch den linken Ventrikel stimulieren können.

Derartige Stimulatoren besitzen typischerweise ein Gehäuse 12, das im Regelfall aus Metall besteht und somit elektrisch leitend ist und als großflächiger Elektrodenpol dienen kann. An der Außenseite des Gehäuses 12 ist typischerweise ein Anschlussgehäuse 14 befestigt, das auch als Header bezeichnet wird. Ein derartiger Header weist typischerweise Kontaktbuchsen zur Aufnahme von Steckkontakten auf. Die Kontaktbuchsen besitzen elektrische Kontakte 16, die über entsprechende Leiter mit einer in dem Gehäuse 12 des Herzstimulators 10 angeordneten Elektronik verbunden sind.

Die Elektrodenleitung 20 stellt im Sinne dieser Erfindung ebenfalls ein implantierbares medizinisches Gerät dar. Am distalen Ende der Elektrodenleitung 20 sind in an sich bekannter Manier Elektrodenpole in Form einer Spitzen- oder Tip-Elektrode 22 und einer in deren Nähe angeordneten Ringelektrode 24 angeordnet. Die Elektrodenpole 22 und 24 sind so ausgebildet, dass sie je nach Funktion eines Herzstimulators, an den die Elektrodenleitung 20 angeschlossen ist, zum Abfühlen elektrischer Potenziale des Herzgewebes (Myokards) dienen oder zur Abgabe elektrischer Signale, beispielsweise zur Abgabe von Stimulationsimpulsen an das sie umgebende Herzgewebe, ausgebildet sind. Fig. 1 zeigt, wie sich die Elektrodenpole, also die Tip-Elektrode 22 und die Ringelektrode 24, im Anwendungsfall die Elektrodenleitung 20, im Apex eines rechten Ventrikels eines Herzens befinden.

Sowohl die Tip-Elektrode 22 als auch die Ringelektrode 24 sind über jeweils wenigstens einen elektrischen Leiter 26 mit einem Steckkontakt 28 am proximalen Ende der Elektrodenleitung 20 elektrisch verbunden. Der Steckkontakt 28 besitzt elektrische Kontakte, die mit den elektrischen Kontakten 16 der Kontaktbuchse im Anschlussgehäuse 14 des implantierbaren Herzstimulators korrespondieren.

Wie weiter unten näher erläutert wird, können die elektrischen Leiter 26 in der Elektrodenleitung 20 in unterschiedlichen Längsabschnitten als annähernd gestreckte Seilzugleiter oder als helixförmig gewendelte Leiter ausgebildet sein. Solche Leiter, die funktionale Elektrodenpole mit elektrischen Kontakten des Steckkontaktes am proximalen Ende der Elektrodenleitung 20 elektrisch leitend verbinden werden im Rahmen dieses Textes auch als Funktionsleiter bezeichnet, da sie beispielsweise der Therapie dienende elektrische Signale von Steckkontakt zum jeweiligen Elektrodenpol übertragen oder abgefühlte elektrische Potentiale repräsentierende Signale vom jeweiligen Elektrodenpol zum Steckkontakt führen und somit der elementaren Funktion des medizinischen Gerätes dienen.

Die elektrischen Funktionsleiter 26, die die Elektrodenpole 22 bzw. 24 mit den elektrischen Kontakten des Steckers 28 der Elektrodenleitung 20 verbinden, sind über den größten Teil ihrer Länge von einer isolierenden Hülle umgeben, so dass ein elektrischer Kontakt zum Gewebe des Herzens gezielt über die Elektrodenpole zustande kommt.

Neben den Elektrodenpolen 22 und 24, die typischerweise der (in diesem Fall ventrikulären) Stimulation des Herzgewebes dienen, weist die Elektrodenleitung 20 auch noch zwei großflächigere Elektrodenpole 30 und 32 auf, die als Defibrillationselektroden dienen und von wenigstens einem blank liegendem helixartig gewendelten Draht gebildet sind.

Es sei darauf hingewiesen, dass die Erfindung im Rahmen dieses Ausführungsbeispiels anhand eines rechtsventrikulären Herzschrittmachers und Defibrillators erläutert wird. Als medizinisches Gerät im Sinne der Erfindung kann grundsätzlich aber beispielsweise auch eine Ablationselektrodenleitung dienen, die im Anwendungsfall ebenfalls bis ins Herz eines Patienten hineinragt und die von einem außerhalb des Patienten angeordneten Gerät gesteuert wird und hierzu mit diesem verbunden ist. Weiterhin können solche Elektrodenleitungen unter fachüblicher Anpassung an die speziellen Erfordernisse des jeweiligen Anwendungsgebiets auch zur Stimulation und Ableitung von Signalen an Nerven, Gehirn, und anderen Organen oder für die Zuleitung von implantierbaren Sensoren Verwendung finden.

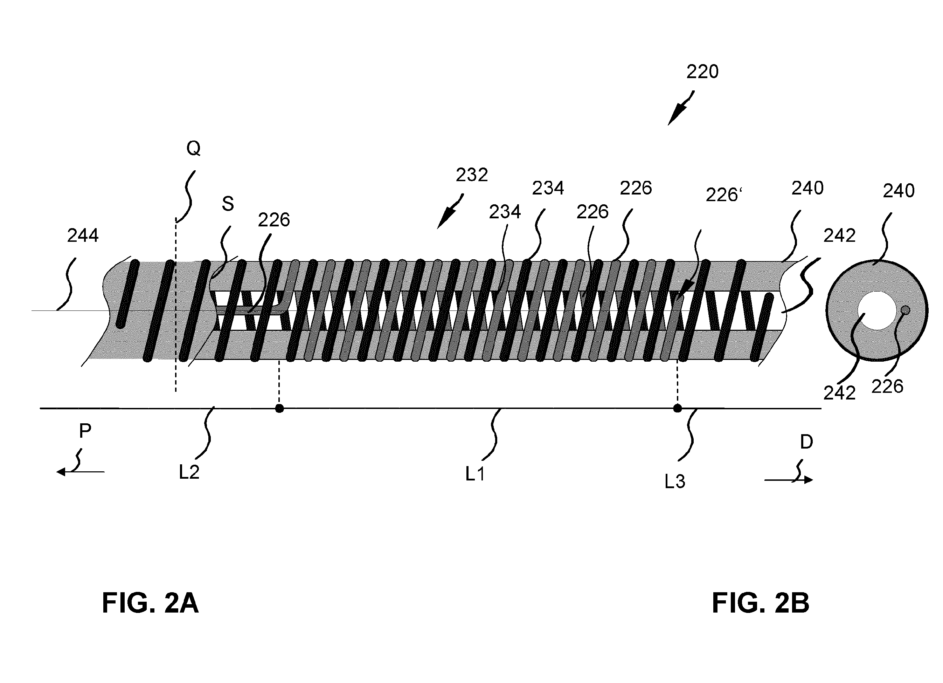

Fig. 2 zeigt in ihrer Teilfigur Fig. 2a eine schematische teilweise Schnittansicht einer Elektrodenleitung und in Fig. 2b eine Querschnittansicht der Elektrodenleitung aus Fig. 2a. Die in Fig. 2a dargestellte Elektrodenleitung 220 ist lediglich in einem Teilabschnitt ihrer Längserstreckung gezeigt. Dieser Teilabschnitt entspricht im vorliegenden Ausführungsbeispiel einem in Fig. 1 im rechten Vorhof des Herzens anzuordnenden Stück der Elektrodenleitung 20, das den Elektrodenpol 32 enthält, kann jedoch ebenso das weiter zum distalen Ende hin liegende Teilstück mit dem Elektrodenpol 30 darstellen. Für die Zwecke der vorliegenden Beschreibung wird ohne Beschränkung der Allgemeinheit angenommen, dass der dem Elektrodenpol 32 entsprechende Teilabschnitt dargestellt ist.

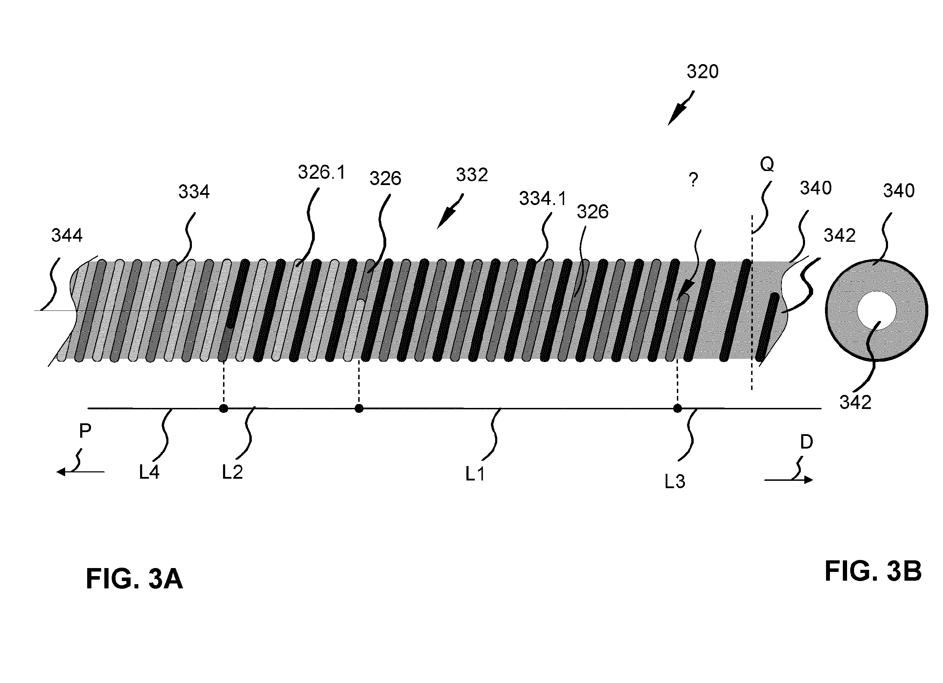

Zur Verdeutlichung dieses Zusammenhangs werden für die Elektrodenleitung und den genannten Elektrodenpol in der vorliegenden Darstellung ähnliche Bezugszeichen wie in Fig. 1 verwendet. Es wird dem dort verwendeten Bezugszeichen lediglich eine "arabische 2" vorangestellt. Wie der weiter unten folgenden Beschreibung der anderen Ausführungsbeispiele der Figuren 3 bis 7 zu entnehmen sein wird, wird dieses Konzept auch dort verwendet, wobei jeweils die der Figurennummerierung entsprechende Ziffer den in Fig. 1 verwendeten Bezugszeichen gleicher funktionaler Elemente vorangestellt wird, um unterschiedliche Varianten der Ausbildung dieser funktionellen Elemente zu kennzeichnen.

Fig. 2A zeigt nun eine teilweise längsgeschnittene Seitenansicht der Elektrodenleitung 220. In der Schnittdarstellung der Fig. 2A ist die Elektrodenleitung 220 jenseits einer Schnittfläche S in einer distalen Richtung D mit einer längsgeschnittenen Ummantelung 240 dargestellt. Dies dient allein der Verdeutlichung des Aufbaus der Elektrodenleitung 220, die tatsächlich eine durchgehend geschlossene Ummantelung 240 aufweist, wie es diesseits der Schnittfläche S in einer proximalen Richtung P sichtbar ist.

Ein Funktionsleiter 226 ist vom hier nicht dargestellten proximalen Ende der Elektrodenleitung 220 bis zum Beginn eines ersten Längsabschnitts L1, der den Elektrodenpol 232 enthält, als Seilzuleitung in der Ummantelung 240 geführt. Die Führung in der Ummantelung 240 ist in der Querschnittdarstellung der Fig. 2B erkennbar, deren Querschnittebene Q in Fig. 2A durch eine gestrichelte Linie gekennzeichnet ist.

Im ersten Längsabschnitt L1 ist der Elektrodenpol 232 als Ganzes betrachtet ringförmig ausgebildet, indem der Funktionsleiter 226 hier nicht mehr als Seilzuleitung, sondern wendelförmig geführt ist. Der Funktionsleiter 226 ist in diesem ersten Längsabschnitt L1 unisoliert und um die Zylinder-Mantelfläche der Ummantelung 240 gewickelt. Im Anschluss an den ersten Längsabschnitt L1 wird der Funktionsleiter 226 wiederum in der Ummantelung 240 geführt, was hier im Einzelnen nicht dargestellt ist. Auch endet der Funktionsleiter 226 an der Stelle 226' in der Fig. 2A, was nicht zwingend notwendig ist. Der Funktionsleiter 226 kann aber auch weitergeführt werden.

Der Funktionsleiter 226 ist im Bereich des Elektrodenpols 232 auf Lücke gewickelt. In diesem Längsabschnitt und sowohl proximal als auch distal daran anschließend ist ein Zusatzleiter 234 wendelförmig um die Ummantelung 240 gewickelt. Im Bereich des Elektrodenpols 232 sind der Funktionsleiter 226 und der Zusatzleiter 234 koradial und auf Lücke gewickelt. Das heißt, der Funktionsleiter 226 und der Zusatzleiter 234 winden sich paarweise nebeneinander um die Ummantelung 240 in einer von der Außenfläche der Ummantelung 240 gebildeten Zylinder-Mantelfläche. Anstelle des einen Zusatzleiters 234 können auch mehrere Zusatzleiter Verwendung finden.

Der Zusatzleiter 234 und der Funktionsleiter 226 sind gegeneinander elektrisch isoliert. Dies kann beispielsweise schon ohne Isolierung der beiden Leiter selbst durch ihren Abstand in Längsrichtung und durch ihre Wickelung auf das isolierende Material der Ummantelung 240 erreicht werden. Für die Ummantelung 240 kann beispielsweise Silikon, aber auch jedes andere für den betreffenden Anwendungszweck aufgrund seiner mechanischen, elektrischen und biologischen Eigenschaften geeignete Material verwendet werden. Alternativ können der Funktionsleiter 226 und der Zusatzleiter 234 zueinander durch eine Leiterisolierung des Zusatzleiters 234 isoliert sein.

Der Zusatzleiter erstreckt sich über den ersten Längsabschnitt L1 hinaus sowohl in der proximalen Richtung P in einem zweiten Längsabschnitt L2 als auch in der distalen Richtung D in einem dritten Längsabschnitt L3.

Die vorliegende Darstellung verzichtet der Einfachheit halber auf hier nicht näher relevante Details, wie beispielsweise zusätzliche mögliche Funktionsleiter, die in einem Lumen 242 der Elektrodenleitung 220 geführt sein können. Die Ummantelung 240 hat die Form eines flexiblen Hohlzylinders, dessen Zylinderachse 244 in Fig. 2A eingezeichnet ist.

Mit der in Fig. 2A und 2B gezeigten Struktur gelingt es, eine elektromagnetische Kopplung zwischen dem Funktionsleiter 226 und dem Zusatzleiter 234 zu erzielen. Diese Kopplung bewirkt insbesondere im Frequenzbereich von elektromagnetischen Radio-frequenz-Wellen, jedoch grundsätzlich bei elektromagnetischen Wellen mit Frequenzen, die deutlich höher als die für Therapiezwecke und für Diagnosezwecke üblichen Frequenzen sind, elektromagnetische Energie vom Funktionswellenleiter 226 in den Zusatzleiter 234 einzukopppeln. Da für Therapiezwecke - wie einleitend erläutert - hochfrequente elektromagnetische Signale auf dem Funktionsleiter schädlich sein können, kann mit Hilfe der Elektrodenleitung 220 ein solcher Schaden vermieden werden und eine unbeeinträchtigte Funktion der Elektrodenleitung auch in Gegenwart hochfrequenter elektromagnetischer Wechselfelder gewährleistet werden. Neben einer induktiven Kopplung besteht auch eine kapazitive Kopplung zwischen dem Funktionsleiter 226 und dem Zusatzleiter 234. Diese kann über die Wahl des isolierenden Materials und den Abstand zwischen den beiden Leitern beeinflusst werden. Je größer der Abstand der beiden Leiter voneinander in der Längsrichtung der Elektrodenleitung 220, desto geringer ist die Einkopplung.

Die Amplitude des eingekoppelten Signals kann durch die Längserstreckung des ersten Längenabschnitts L1 beeinflusst werden. Je größer die Koppellänge, desto größer ist die Amplitude des eingekoppelten Signals bis zum Erreichen einer Sättigung.

Fig. 3 zeigt in ihrer Teilfigur Fig. 3A eine schematische Seitenansicht einer Elektrodenleitung und Fig. 3b eine Querschnittsansicht der Elektrodenleitung aus Fig. 3a. Die Elektrodenleitung der Figuren 3a und 3b ähnelt in vielen Merkmalen der Elektrodenleitung 220 der Figuren 2a und 2b. Die nachfolgende Beschreibung konzentriert sich daher allein auf die Unterschiede der beiden Ausführungsbeispiele.

Anders als im Beispiel der Figuren 2A und 2B ist im vorliegenden Ausführungsbeispiel die Funktionsleitung 326 auch über den ersten Längsabschnitt L1 hinaus wendelförmig um die Umhüllung 340 gewickelt, und zwar erstreckt sich der Abschnitt mit wendelförmiger Führung in die proximale Richtung P auch über Längsabschnitte L2 und L4 hinweg. Dabei ist die Funktionsleitung in den Längsabschnitten L1, L2 und L4 durchgehend koradial und auf Lücke gewickelt mit dem Zusatzleiter 334 kombiniert.

Im vorliegenden Ausführungsbeispiel ist der Zusatzleiter, der auch als Verlustwendel bezeichnet werden könnte, in einigen Längsabschnitten unisoliert. Beispielsweise ist der Zusatzleiter 334 im Längsabschnitt L4 unisoliert, während er daran anschließend im Längsabschnitt L2 sowie in den daran anschließenden Längsabschnitten L1 und L3 isoliert ausgeführt ist. Die in den letztgenannten drei Längsabschnitten vorgesehene Leiterisolierung kann alternativ auch fortgelassen werden, wenn der Zusatzleiter ausreichend vom Funktionsleiter 326 durch das isolierende Material der Ummantelung 340 isoliert ist.

Der Funktionsleiter 326 ist, wie im vorhergehenden Ausführungsbeispiel, im ersten Längsabschnitt L1 unisoliert, um seine therapeutische oder diagnostische Funktion zu ermöglichen. Die Elektrodenleitung 332 des vorliegende n Ausführungsbeispiels ermöglicht aufgrund ihrer abweichenden Merkmale eine erhöhte induktive und kapazitive Kopplung zwischen dem Funktionsleiter 326 und dem Zusatzleiter 334. Darüber hinaus ermöglicht sie eine Abgabe von elektromagnetischer Energie außerhalb des ersten Längsbereiches L1 des funktionellen Elektrodenpols 332, nämlich in den Längsbereichen L4, in denen der Zusatzleiter 334 nicht gegen das Körpergewebe isoliert ist.

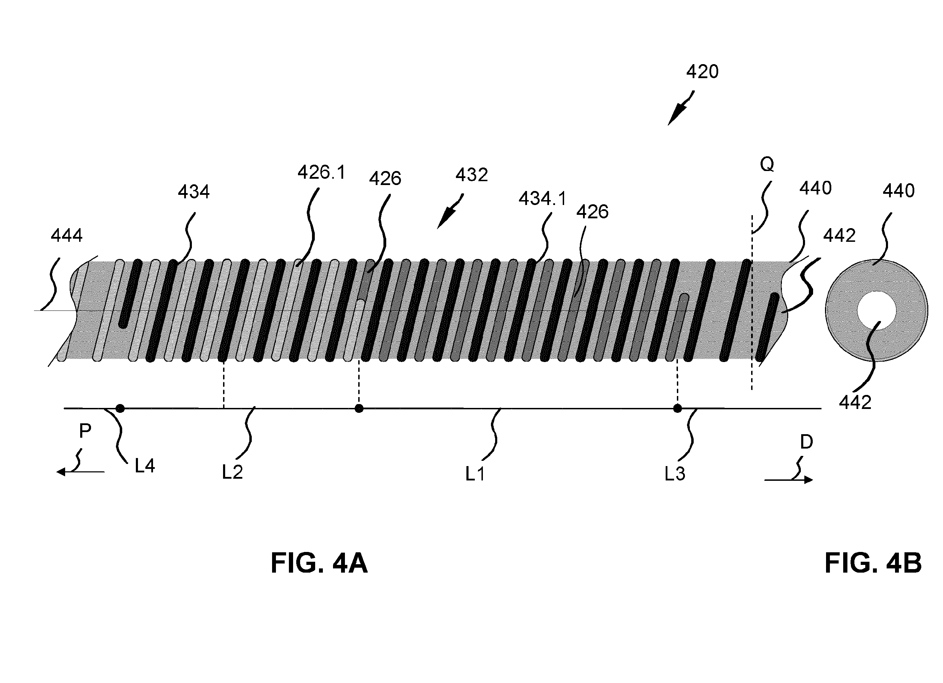

Fig. 4 zeigt in ihrer Teilfigur Fig. 4a eine schematische Seitenansicht einer Elektrodenleitung und in Fig. 4B eine Querschnittsansicht der Elektrodenleitung aus Fig. 4A. Die Elektrodenleitung 420 des vorliegenden Ausführungsbeispiels ähnelt dem der Fig. 3 und unterscheidet sich allein darin, dass der Zusatzleiter 434 in allen Längsabschnitten, in denen er koradial mit dem Funktionsleiter 426 geführt ist, isoliert ist. Darüber hinaus erstreckt sich der Zusatzleiter 434 im vorliegenden Ausführungsbeispiel längs der Elektrodenleitung 420 in proximaler Richtung nur bis kurz hinter dem Beginn des vierten Längsabschnitts L4 wendelförmig um die Außenfläche der Ummantelung 440 herum. Er hat damit zwar weiterhin eine größere Längserstreckung als der funktionelle Elektrodenpol 432, verläuft jedoch außerhalb der dargestellten Längsabschnitte innerhalb der Ummantelung 440. Dieses Design reduziert die Kopplung zwischen dem Funktionsleiter 426 und dem Zusatzleiter 434 und begrenzt sie auf den Elektrodenpol 432 und dessen unmittelbares Umfeld.

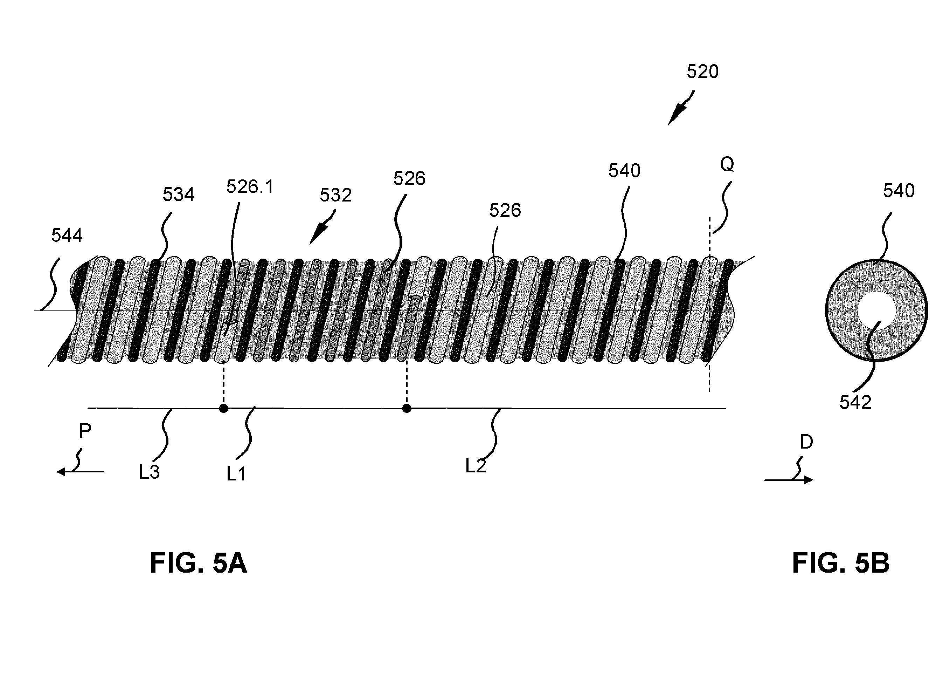

Fig. 5 zeigt in ihrer Teilfigur Fig. 5A eine schematische Seitenansicht einer Elektrodenleitung und in Fig. 5B eine Querschnittsansicht der Elektrodenleitung aus Fig. 5A. Die Elektrodenleitung 520 des vorliegenden Ausführungsbeispiels hat eine Funktionsleitung 526, die in Längsabschnitten außerhalb des Elektrodenpols 532 isoliert ist. Der Zusatzleiter 534 ist durchgängig isoliert. Der Funktionsleiter 526 und der Zusatzleiter 534 haben im vorliegenden Ausführungsbeispiel unterschiedliche Dicken, also Leiter- oder Drahtstärken. Darüber hinaus kann alternativ oder zusätzlich die Isolierung eines der beiden Leiter 526 und 534 eine andere Stärke, also Dicke, aufweisen als die Isolierung des anderen Leiters. Im vorliegenden Beispiel hat der Funktionsleiter 526 eine dickere Isolierung als der Zusatzleiter 534 und zusätzlich eine höhere Leiterdicke, also Drahtstärke, ohne Berücksichtigung der ihn umgebenden Isolierung. Der Außendurchmesser des abisolierten Funktionsleiters im ersten Längsbereich L1 der Funktionselektrode 532 ähnelt absichtlich dem Außendurchmesser des in diesem Bereich isolierten Zusatzleiters 534.

Diese Ausführungsform hat den Vorteil, dass der Funktionselektrodenpol 532 durch Abisolieren des Funktionsleiters leicht hergestellt werden kann, ohne die Isolierung des Zusatzleiters 534 zu beschädigen.

In anderen - hier nicht dargestellten - Längsabschnitten ist allein der Zusatzleiter 534 unisoliert ist und der Funktionsleiter 526 isoliert. Auf diese Weise können Bereiche des Zusatzleiters 534 direkt mit dem Gewebe kontaktiert werden, um eine Wärmeableitung über einen passiven, nicht für therapeutische oder diagnostische Zwecke genutzten Elektrodenbereichs zu ermöglichen. Das Ausführungsbeispiel ermöglicht im Rahmen der Herstellung der Elektrodenleitung eine erleichterte selektive Abisolierung zur Herstellung entweder eines Funktionselektrodenpols oder des passiven Elektrodenbereichs.

Die Ausführungsbeispiele der Figuren 2 bis 5 haben auf Lücke gewickelte Funktions- und Zusatzleiter. Der Vorteil dieser Wicklung auf Lücke besteht in ihrer besonders hohen Kompressibilität.

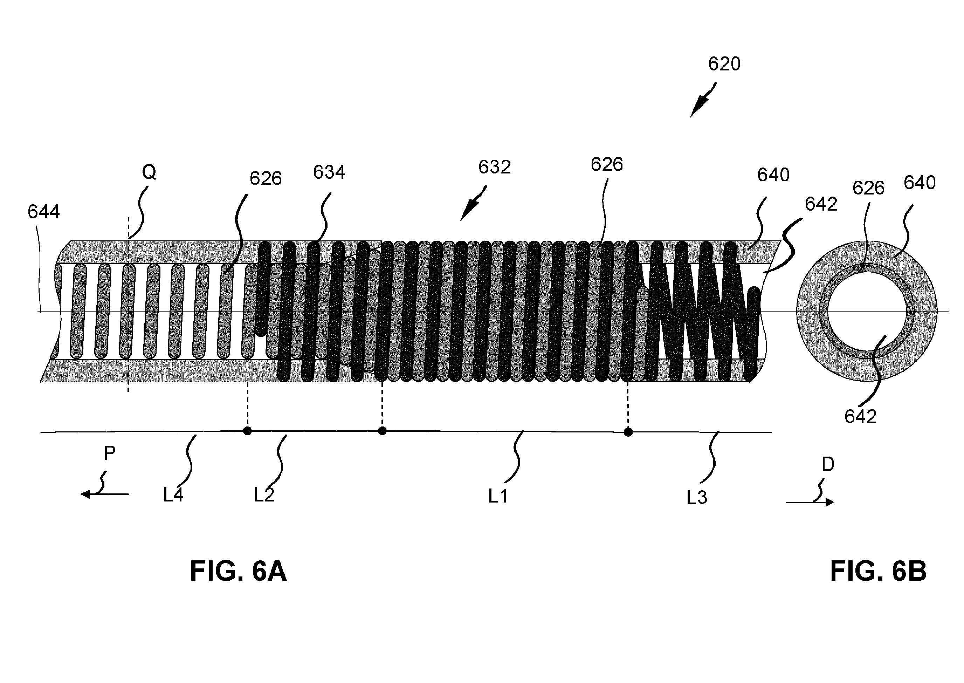

Fig. 6 zeigt in ihrer Teilfigur Fig. 6A eine schematische Schnittansicht einer Elektrodenleitung und in Fig. 6B eine Querschnittsansicht der Elektrodenleitung aus Fig. 6A. Anders als in den zuvor gezeigten und zuvor erläuterten Ausführungsbeispielen hat die Elektrodenleitung 620 im Bereich des Elektrodenpols 632 eine Unterbrechung der Ummantelung 640. In dem betreffenden Längsabschnitt L1 sind der Funktionsleiter 626 und der Zusatzleiter 635 koradial und wendelförmig auf Block gewickelt. Der Funktionsleiter 626 und der Zusatzleiter 634 sind gegeneinander isoliert. Der Zusatzleiter 634 erstreckt sich über den Längsabschnitt L1 des als Ringelektrode ausgeführten Elektrodenpols 632 hinaus proximal auch im Längsabschnitt L2 und distal auch im Längsabschnitt L3. Der Funktionsleiter 626 ist im vorliegenden Ausführungsbeispiel durchgehend wendelförmig geführt und erstreckt sich im Längsabschnitt L4, also von einem proximalen Elektrodenanschluss bis hin zum Bereich des Elektrodenpols 632, im Lumen 642 der Ummantelung 640. Dieses Ausführungsbeispiel hat insbesondere im Längsabschnitt L1 des Elektrodenpols 632 eine besonders starke induktive und kapazitive Kopplung zwischen dem Funktionsleiter 626 und dem Zusatzleiter 634.

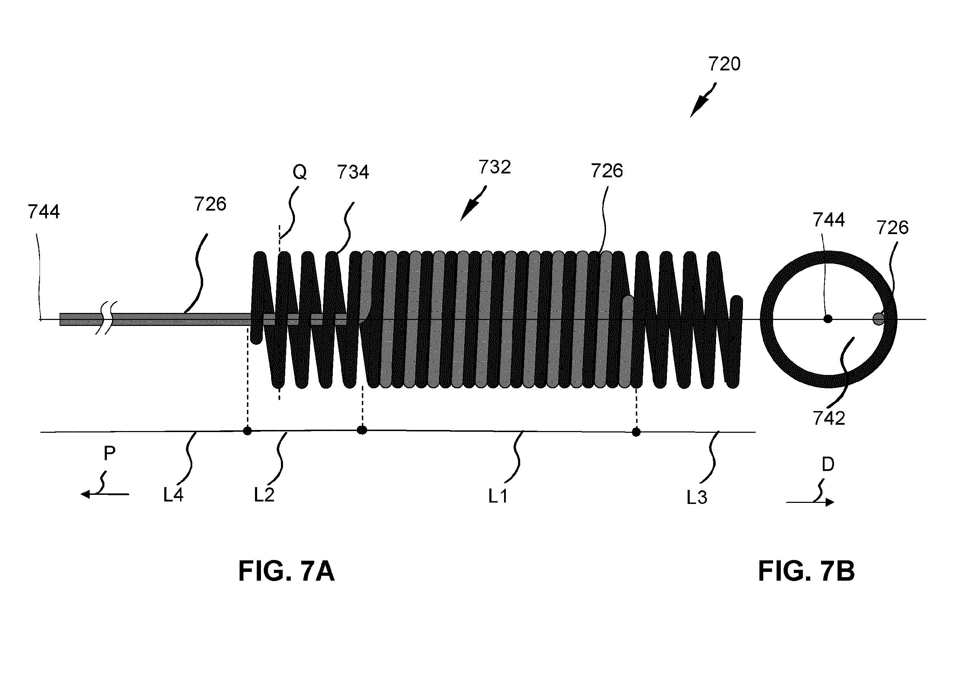

Fig. 7 zeigt in ihrer Teilfigur Fig. 7A eine schematische teilweise Seitenansicht einer Elektrodenleitung und in Fig. 7B eine Querschnittsansicht der Elektrodenleitung aus Fig. 7A, wobei jeweils eine Ummantelung vorhanden ist, die hier aber nicht dargestellt wird. Das Ausführungsbeispiel der Fig. 7A und Fig. 7B unterscheidet sich von dem der Fig. 6 alleine dadurch, dass der Funktionsleiter 726 bis zum Längsabschnitt L1 des Elektrodenpols 732 als Seilzuleitung geführt ist. Der Funktionsleiter ist in diesen Längsabschnitten L2 und L4, in denen er als Seilzuleitung ausgebildet ist, im Lumen der Elektrodenleitung 720 geführt, und zwar nicht auf einer Mittelachse des von dem Elektrodenpol 732 gebildeten Hohlzylinders.

## Patentansprüche

1. Temporär oder permanent implantierbares medizinisches Gerät (10), mit mindestens einer langgestreckten elektrischen Leitung (20, 220),
- die einen Funktionsleiter (26, 226, 326, 426, 526, 626, 726) enthält, welcher mit einem Elektrodenpol (22, 24, 30, 32, 232, 332, 432, 532, 632, 732) zur Abgabe von Therapiesignalen oder zur Erfassung von Diagnosesignalen verbunden ist, wobei der Funktionsleiter (26, 226, 326, 426, 526, 626, 726) oder der Elektrodenpol (22, 24, 30, 32, 232, 332, 432, 532, 632, 732), oder sowohl der Funktionsleiter 26, 226, 326, 426, 526, 626, 726) als auch der Elektrodenpol (22, 24, 30, 32, 232, 332, 432, 532, 632, 732) in einem ersten Längsabschnitt (L1) ringförmig ausgebildet ist; und
- die mindestens einen zweiten elektrischen Leiter (234, 334, 434, 534, 634, 635) enthält, der in dem ersten Längsabschnitt (L1) wendelförmig geführt ist, derart, dass im ersten Leiter führbare elektromagnetische Radiofrequenz-Wellen zumindest teilweise im ersten Längsabschnitt (L1) in den zweiten elektrischen Leiter einkoppelbar sind.

2. Medizinisches Gerät nach Anspruch 1, bei dem der ringförmig ausgebildete Elektrodenpol (22, 30, 32, 232, 332, 432, 532, 632, 732) von dem im ersten Längsabschnitt (L1) unisoliert und wendelförmig geführten Funktionsleiter (26, 226, 326, 426, 526, 626, 726) gebildet ist.

3. Medizinisches Gerät nach Anspruch 1 oder 2, bei dem der zweite elektrische Leiter (234, 334, 434, 534, 634, 635) keinen Funktionsleiter (26, 226, 326, 426, 526, 626, 726), sondern einen oder mehrere Zusatzleiter (234, 334, 434, 534, 634, 635) enthält.

4. Medizinisches Gerät nach einem der vorstehenden Ansprüche, bei dem der Funktionsleiter (26, 226, 326, 426, 526, 626, 726) und der zweite elektrische Leiter (234, 334, 434, 534, 634, 635) auf einer gemeinsamen, gedachten Zylindermantel-Fläche wendelförmig geführt sind.

5. Medizinisches Gerät nach Anspruch 4 , bei dem der Funktionsleiter (26, 226, 326, 426, 526, 626, 726) und der zweite elektrische Leiter (234, 334, 434, 534, 634, 635) im ersten Längsabschnitt L1 als von einander elektrisch isoliertes Leiterpaar koradial geführt sind.

6. Medizinisches Gerät nach einem der Ansprüche 2 bis 5, bei dem der Funktionsleiter (26, 226, 326, 426, 526, 626, 726) und der zweite elektrische Leiter (234, 334, 434, 534, 634, 635) im ersten Längsabschnitt (L1) auf Block gewickelt sind.

7. Medizinisches Gerät nach einem der Ansprüche 2 bis 5, bei dem der Funktionsleiter (26, 226, 326, 426, 526, 626, 726) und der zweite elektrische Leiter (234, 334, 434, 534, 634, 635) im ersten Längsabschnitt (L1) auf Lücke gewickelt sind.

8. Medizinisches Gerät nach einem der Ansprüche 2 bis 7, bei dem der zweite elektrische Leiter (234, 334, 434, 534, 634, 635) im ersten Längsabschnitt (L1) von einer Leiterisolierung umgeben ist.

9. Medizinisches Gerät nach einem der Ansprüche 2 bis 8, bei dem der zweite elektrische Leiter (234, 334, 434, 534, 634, 635) einen anderen Durchmesser hat als der Funktionsleiter (26, 226, 326, 426, 526, 626, 726).

10. Medizinisches Gerät nach einem der Ansprüche 2 bis 9, bei dem der Funktionsleiter und der zweite elektrische Leiter im ersten Längsabschnitt in eine elektrisch isolierende Ummantelung des ersten elektrischen Leiters eingebettet sind.

11. Medizinisches Gerät nach einem der Ansprüche 2 bis 10, bei dem der zweite elektrische Leiter (234, 334, 434, 534, 634, 635) sich in proximaler oder distaler Richtung oder sowohl in proximaler als auch in distaler Richtung wendelförmig gewickelt über den ersten Längsabschnitt (L1) hinaus erstreckt.

12. Medizinisches Gerät nach Anspruch 11, bei dem der zweite elektrische Leiter (234, 334, 434, 534, 634, 635) in Längsabschnitten außerhalb des ersten Längsabschnitts (L1) auf eine Ummantelung der des ersten elektrischen Leiters (26, 226, 326, 426, 526, 626, 726) gewickelt ist.

13. Medizinisches Gerät nach einem der vorstehenden Ansprüche, bei dem der Funktionsleiter (26, 226, 326, 426, 526, 626, 726) außerhalb des ersten Längsabschnitts (L1) als Seilzuleitung ausgebildet ist.

14. Medizinisches Gerät nach einem der vorstehenden Ansprüche, mit einer hohlzylindrischen Ummantelung, die im Inneren ein Lumen aufweist, in dem eine Innenwendel des ersten elektrischen Leiters (26, 226, 326, 426, 526, 626, 726) geführt ist.

15. Medizinisches Gerät nach Anspruch 13 und 14, bei dem die Seilzuleitung außerhalb des ersten Längsabschnitts (L1) in die Ummantelung eingebettet ist.
